# FASCICULE DE BREVET EUROPEEN

(11) **EP 1 254 635 B1**
(45) Date de publication et mention de la délivrance du brevet: **19.03.2008**
(21) Numéro de dépôt: 02291124.2
(22) Date de dépôt: 03.05.2002
(51) Int. Cl.: A61B 17/04

(54) **Instrument permettant de fermer par suture sous-cutanée un orifice réalisé dans la paroi abdominale d'un patient**
Subkutane Nähvorrichtung zum Schliessen einer Öffnung der Bauchdecke eines Patienten
Subcutaneous suturing device for closing abdominal wall port of a patient

(30) Priorité: 04.05.2001 FR 0106022
(43) Date de publication de la demande: 06.11.2002
(73) Titulaire: Navarro, Francis, 30000 Nimes (FR); Domergue, Jacques, 34000 Montpellier (FR)
(72) Inventeur: Navarro, Francis, 30000 Nimes (FR); Domergue, Jacques, 34000 Montpellier (FR)
(74) Mandataire: Thinat, Michel

(56) Documents cités:
- US-A- 5 320 632
- US-A- 5 374 275
- US-A- 5 741 279
- US-A- 5 860 991

## Description

La présente invention concerne un instrument permettant de fermer par suture sous-cutanée un orifice réalisé dans la paroi abdominale d'un patient.

Lorsqu'une intervention chirurgicale est effectuée par laparoscopie, l'abdomen du patient est gonflé par un gaz et le chirurgien opère par l'intermédiaire de trocarts.

Un trocart est un dispositif cylindrique qui traverse la paroi abdominale du patient et au travers duquel sont introduits le ou les instruments permettant d'effectuer l'intervention chirurgicale.

Pour mettre en place ces trocarts cylindriques, qui peuvent avoir un diamètre de 5 à 15 mm, il est nécessaire d'effectuer un incision cutanée et le trocart est introduit dans l'abdomen par son extrémité munie d'une pointe et de moyens de protection. En fin d'intervention chirurgicale, le trocart est retiré et l'orifice par lequel il a été introduit, ayant une longueur d'environ 12 mm, doit être refermé pour une bonne cicatrisation. Pour des orifices de passage de trocarts d'une longueur supérieure à 10 mm, il est nécessaire d'effectuer leur fermeture par suture du plan sous-cutané, c'est-à-dire de l'aponévrose et du muscle qui est la partie rigide résistante. Jusqu'à présent, de tels orifices étaient fermés en mettant un point de suture au niveau de l'aponévrose superficielle ou profonde suivant l'épaisseur des parois abdominales des patients. La difficulté majeure pour réaliser un tel point de suture est d'écarter la peau, partie la plus superficielle, et d'aller chercher le plan musculaire et l'aponévrose qui est le tissu le plus rigide sur lequel doit être mis le point de suture qui sera noué de l'extérieur vers l'intérieur. Les chirurgiens ont souvent des difficultés à fermer le tissu profond qu'est l'aponévrose et, parfois, ce tissu n'est pas refermé. En outre, certaines complications peuvent s'ensuivre, telles la non cicatrisation et la non fermeture de l'orifice profond, pouvant causer des éventrations. Une autre complication peut survenir concernant l'incarcération d'éléments intra-abdominaux tels que par exemple l'intestin grêle à l'intérieur de cet orifice avec le risque d'entraîner des occlusions intestinales ou d'autres complications digestives.

US 5 320 632 décrit un instrument chirurgical comportant les caractéristiques énoncées dans le préambule de la revendication 1.

Selon cet instrument connu, l'organe de support des aiguilles est constitué de deux parties pouvant être écartées l'une de l'autre de part et d'autre de l'extrémité de la canule sous l'action du piston et à l'encontre de la force de rappel d'un ressort s'étendant transversalement au travers de la canule et dont les deux extrémités sont reliées respectivement aux deux parties de support des aiguilles.

La présente invention a pour but de résoudre les problèmes ci-dessus en proposant un instrument permettant de fermer efficacement un orifice réalisé dans la paroi abdominale d'un patient pour permettre une bonne cicatrisation de cet orifice.

A cet effet, l'invention propose un instrument comprenant les caractéristiques énoncées dans la partie caractérisante de la revendication 1.

De préférence, le piston est une tige rigide centrale à coulissement guidé dans le moyen d'extraction et dont l'extrémité inférieure est reliée à l'organe de support des aiguilles et la partie d'extrémité supérieure traverse une paroi transversale supérieure de fermeture de la canule, la tige pouvant être axialement retenue dans la canule par un moyen du verrouillage manuellement déverrouillable de façon à exercer sur l'organe de support une force axiale de rétraction des aiguilles dans la canule pour permettre l'introduction de celle-ci dans l'orifice, l'opérateur pouvant ensuite déverrouiller le moyen de verrouillage pour déplacer la tige dans un sens provoquant le déploiement de l'organe de support et la sortie des aiguilles de la canule.

Le moyen d'extraction comprend une partie supérieure cylindrique de coulissement dans la canule, une partie d'extrémité inférieure cylindrique de plus petit diamètre dans laquelle est montée à coulissement la partie d'extrémité inférieure de la tige et une partie intermédiaire de liaison constituée notamment de deux parois obliques convergeant vers la partie d'extrémité inférieure et comprenant chacune une fenêtre longitudinale permettant, en position basse du moyen d'extraction, le passage de l'aiguille correspondante à sa position sortie en saillie de la canule, chaque fenêtre se terminant au-dessus de la partie inférieure du moyen d'extraction par deux bords en forme de V dans lequel peut s'accrocher l'extrémité en forme de crochet de l'aiguille lors du déplacement du moyen d'extraction vers la partie supérieure de la canule.

Le moyen d'extraction comprend en outre deux pattes externes de préhension diamétralement opposées solidaires de la partie supérieure de ce moyen en faisant radialement saillie au travers respectivement de deux fenêtres longitudinales de la paroi latérale de la canule situées au-dessus des fenêtres de passage des aiguilles et un ressort est monté précontraint entre le moyen d'extraction et la canule pour rappeler le moyen d'extraction à sa position basse.

Les aiguilles sont courbées et situées respectivement dans deux plans parallèles au plan médian longitudinal de la canule, disposés de part et d'autre de ce plan médian à égale distance de celui-ci de façon à permettre aux aiguilles d'occuper leur position rentrée en se croisant dans la partie d'extrémité de la canule.

Les aiguilles sont montées amoviblement par leurs extrémités opposées à celles en forme de crochet respectivement dans deux embases à section transversale circulaire, triangulaire, ou autre, et solidaires des extrémités de l'organe de support, l'axe de chaque embase étant incliné relativement au plan de support d'extrémité correspondante de l'organe de support pour faciliter l'extraction des aiguilles lors de la remontée du moyen d'extraction dans la canule.

Les aiguilles sont introduites dans la paroi abdominale, une fois occupant leur position sortie de la canule en dessous de cette paroi, par traction sur la canule pour les faire pénétrer dans la paroi et ensuite en exerçant sur le piston un effort dans un sens pour rétracter les aiguilles vers la canule et faire pénétrer les extrémités due celle-ci dans l'orifice et dans la canule au travers des deux fenêtres diamétralement opposées de celle-ci.

Les deux plans contenant respectivement les deux aiguilles sont situés de part et d'autre de la tige du piston.

Selon un mode de réalisation, l'organe de support est constitué par une lame flexible portant à ses extrémités les deux aiguilles et maintenue dans la partie inférieure de la canule perpendiculairement au plan médian longitudinal de cette dernière par deux paires d'axes de support parallèles à la lame, disposées de part et d'autre du plan médian transversal de la lame symétriquement à celui-ci et solidaires du corps de la canule perpendiculairement à son plan médian longitudinal, les deux axes de chaque paire étant situés de chaque côté de la lame à proximité immédiate de celle-ci pour modifier par flexion le rayon de courbure de la lame par glissement de celle-ci entre les axes de support sous l'action de la tige du piston, dont l'extrémité inférieure est solidaire du centre géométrique de la lame, et déplacer vers ce centre vers le bas ou vers le haut de la canule pour sortir ou rentrer, et ultérieurement rentrer partiellement, les aiguilles de ou dans la canule.

Selon un autre mode de réalisation, l'organe de support est constitué par deux bras montés pivotants sur un axe central commun solidaire du corps de la canule en partie inférieure de celle-ci et s'étendant perpendiculairement au plan médian longitudinal de la canule, les extrémités opposées des deux bras portant les aiguilles, et le piston commande le pivotement simultané des deux bras en le déplaçant vers le bas ou vers le haut pour sortir ou rentrer les aiguilles de ou dans la canule par l'intermédiaire de deux bras de traction reliés d'une part l'un à l'autre à l'extrémité inférieure de la tige du piston par un axe d'articulation solidaire du piston parallèlement à l'axe de pivotement des bras de support des aiguilles et d'autre part à leurs extrémités opposées de façon articulée respectivement aux deux bras de support, au voisinage des aiguilles.

Le moyen de verrouillage de la tige à sa position de ploiement de l'organe de support comprend deux ergots diamétralement opposés solidaires de la partie supérieure de la tige en faisant radialement saillie de celle-ci et venant en appui sous ou sur la paroi transversale de fermeture de la canule suivant que l'organe de support est constitué par la lame flexible ou les deux bras pivotants, cette paroi comportant un trou oblong défini de part et d'autre de l'orifice central de passage de la tige et permettant le passage des deux ergots à travers celui-ci par rotation de la partie supérieure de la tige relativement à sa partie inférieure pour déverrouiller la tige et la déplacer axialement dans la canule.

L'invention sera mieux comprise, et d'autres buts, caractéristiques, détails et avantages de celle-ci apparaîtront plus clairement dans la description explicative qui va suivre faite en référence aux dessins schématiques annexés donnés uniquement à titre d'exemple illustrant deux modes de réalisation de l'invention et dans lesquels :
- la figure 1 est une vue en perspective éclatée de l'instrument de l'invention introduit au travers d'une paroi abdominale d'un patient ;
- les figures 2 à 8 représentent l'instrument de la figure 1 à différentes phases de fonctionnement pour fermer par suture sous-cutanée un orifice réalisé dans la paroi abdominale du patient ;
- la figure 9 représente un autre mode de réalisation de l'organe de support des aiguilles faisant partie de l'instrument de fermeture de l'orifice et occupant sa position déployée ;
- la figure 10 est une vue de l'organe de support de la figure 9 en position ployée ; et
- la figure 11 est une vue de dessus suivant la flèche XI de la figure 9.

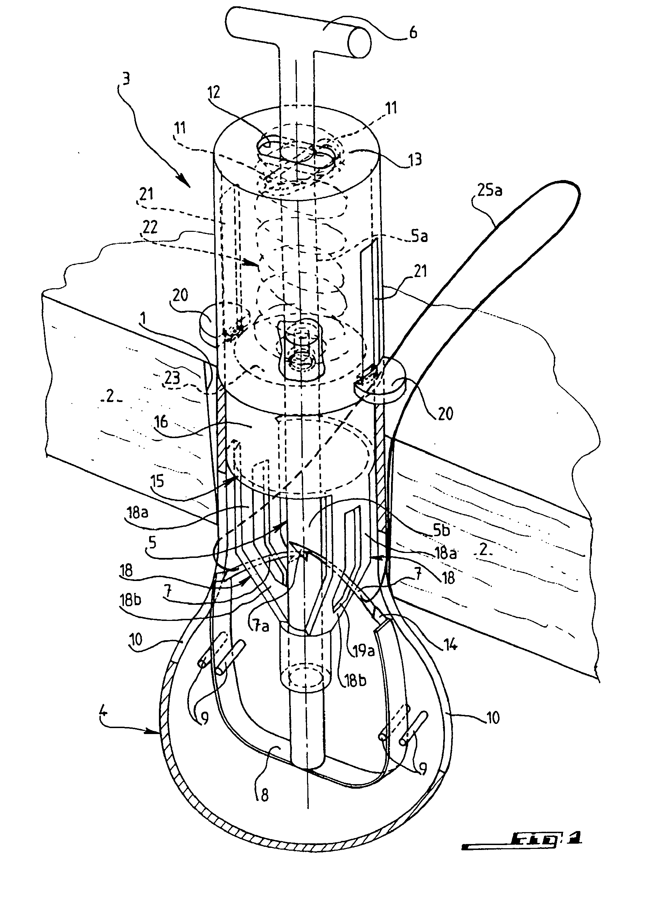

En se reportant aux figures 1 à 8, l'instrument de l'invention est destiné à permettre la fermeture par suture sous-cutanée d'un orifice 1 réalisé à travers une paroi abdominale 2 d'un patient pour le passage d'un trocart ayant auparavant servi à une intervention chirurgicale par laparoscopie.

L'instrument comprend une canule rigide cylindrique 3, par exemple en métal, pouvant être partiellement introduite dans l'orifice 1 immédiatement après retrait du trocart une fois l'intervention chirurgicale terminée, c'est-à-dire à un moment où l'abdomen du patient est toujours gonflé par un gaz approprié.

La figure 1 représente l'instrument à sa position initiale d'introduction à travers l'orifice 1 de la paroi abdominale 2 et à laquelle la partie d'extrémité inférieure 4 de l'instrument est située dans l'abdomen en dessous de la paroi 2.

L'instrument comprend en outre un piston central 5 en forme de tige rigide cylindrique s'étendant coaxialement dans la canule 3 et pouvant être actionné manuellement de l'extérieur de la canule 3 par l'intermédiaire d'une poignée externe de préhension 6 pour coulisser axialement de façon guidée relativement à la canule 3 comme on le verra ultérieurement.

L'instrument comprend en outre deux aiguilles 7 amoviblement fixées aux extrémités diamétralement opposées d'un organe de support 8 constitué par une lame flexible supportée dans la partie d'extrémité inférieure 4 de la canule 3. A cet effet, la lame flexible 8 est maintenue dans la partie 4 perpendiculairement au plan médian longitudinal de la canule 3 par deux paires d'axes de support 9 parallèles à la lame 8 et disposées de part et d'autre du plan médian transversal de cette lame symétriquement à ce plan. Les axes de support 9 sont solidaires du corps de la partie inférieure 4 de la canule 3 perpendiculairement au plan médian longitudinal de la canule et les deux axes 9 de chaque paire sont situés de chaque côté de la lame 8 à proximité immédiate de celle-ci en étant décalés l'un par rapport à l'autre le long de la lame 8.

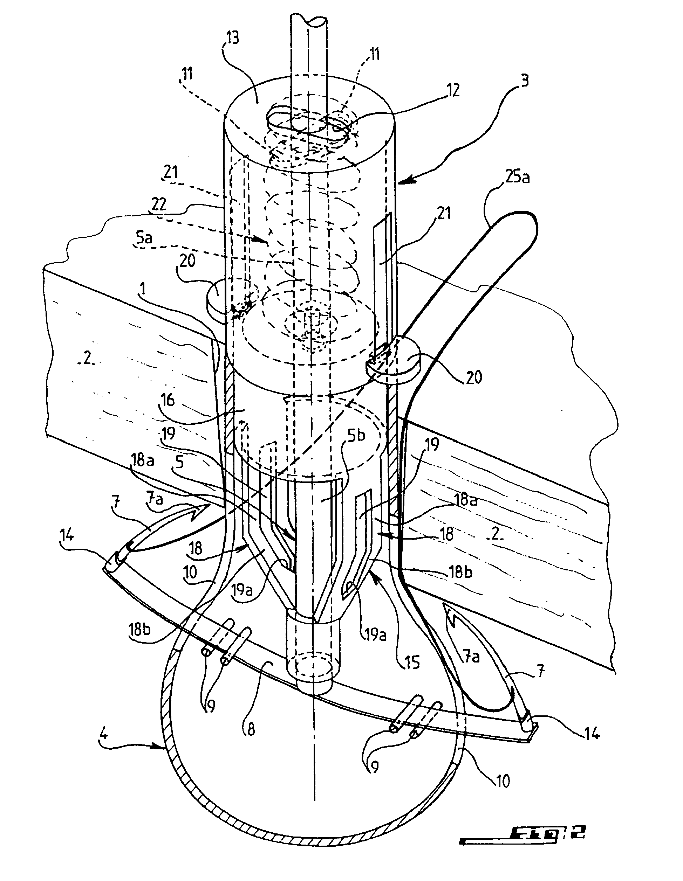

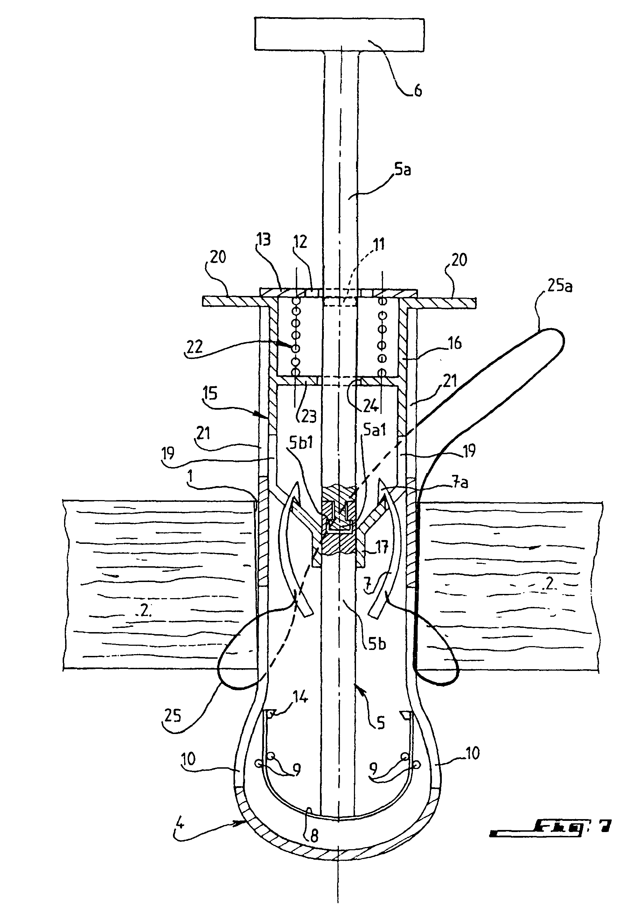

L'extrémité inférieure de la tige de piston 5 est solidaire, par tout moyen approprié, tel qu'une vis de fixation, du centre géométrique de la lame de support 8 de façon qu'un déplacement vers le haut de la canule 3 de la tige 5 provoque une déformation élastique de la lame 8 par glissement de celle-ci entre les paires d'axes de support 9 pour sortir les parties d'extrémité opposées de la lame 8 au travers respectivement de deux fenêtres rectangulaires longitudinales 10 diamétralement opposées réalisées à travers la paroi latérale du corps de la partie d'extrémité inférieure 4 de la canule 3, de façon que les deux aiguilles 7 fassent complètement saillie en dehors de la partie d'extrémité 4 comme représenté en figure 2. Un déplacement vers le bas de la tige de piston 5 à partir de sa position représentée en figure 2 provoque une déformation par flexion de la lame 8 vers l'extrémité inférieure de la canule 3 et le coulissement de la lame 8 entre les deux paires d'axes de support 9 qui déforment la lame 8 de façon que ses parties d'extrémité puissent à nouveau entrer dans la partie d'extrémité 4 de la canule 3 au travers des deux fenêtres 10 comme représenté en figure 7.

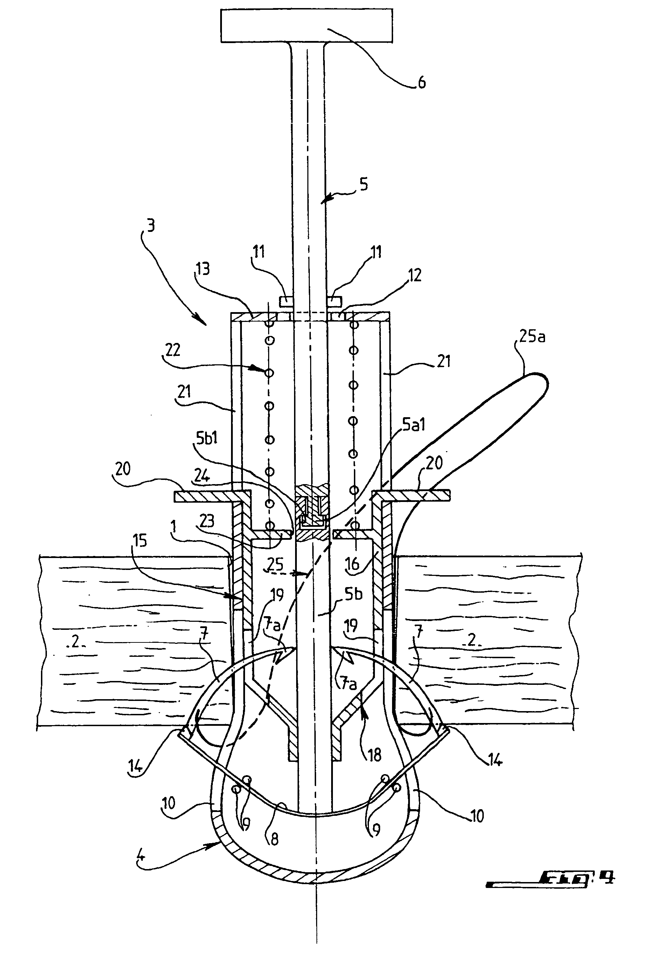

La tige de piston 5 est verrouillée à sa position initiale représentée en figure 1 par un moyen de verrouillage déverrouillable manuellement et constitué de deux ergots 11 solidaires de la tige 5 en partie supérieure 5a de celle-ci en faisant saillie de celle-ci et diamétralement opposés. Les deux ergots 11 peuvent occuper une position transversale à une lumière 12 réalisée au travers de la paroi circulaire 13 de fermeture de l'extrémité supérieure de la canule 3 et sous laquelle sont en appui les deux ergots 11 sous l'action de la force élastique de réaction exercée par la lame 8 sur la tige 5. La partie supérieure 5a de la tige 5 peut être tournée à l'aide de la poignée 6 relativement à la partie inférieure 5b de celle-ci d'environ 90° pour amener les deux ergots 11 en aplomb de la lumière 12 pour déverrouiller la tige 5 de la canule 3 et permettre d'exercer une traction sur la tige 5 provoquant le déplacement de la lame 8 dans un sens amenant les aiguilles 7 à sortir de la partie d'extrémité 4 au travers des deux fenêtres 10. Comme cela ressort notamment de la figure 4, la partie supérieure 5a de la tige 5 comporte à son extrémité une tête circulaire 5a1 de plus petit diamètre engagée dans un lamage de forme conjuguée 5b1 réalisé à l'extrémité de la partie inférieure 5b de la tige 5 à l'opposé de la lame 8, de façon à permettre une rotation de la partie supérieure 5a relativement à la partie inférieure 5b de la tige 5. La tête 5a1 peut être constituée par une tête de vis dont la partie filetée est bloquée par vissage dans l'extrémité inférieure de la partie 5a de la tige 5 coaxialement à celle-ci.

Les aiguilles 7 sont montées chacune à l'extrémité correspondante de la lame 8 dans une embase 14 solidaire de l'extrémité de la lame 8 et ayant une section transversale qui peut être circulaire, triangulaire, ou autres. L'axe de chaque embase 14 est incliné relativement au plan de support d'extrémité correspondante de la lame 8 pour faciliter l'extraction de l'aiguille 7 comme on le verra ultérieurement. En variante, toujours pour faciliter l'extraction de chaque aiguille 7, chaque embase 14 peut avoir son axe perpendiculaire au plan d'extrémité correspondante de la lame 8, mais posséder une face d'extrémité libre inclinée relativement à ce plan d'extrémité de façon que l'extrémité de l'aiguille 7 s'engageant dans l'embase 14 ait une partie d'épaulement venant en appui sur cette face inclinée.

Les aiguilles 7 sont identiques, courbées et dirigées l'une vers l'autre en étant situées respectivement dans deux plans parallèles au plan médian longitudinal de la canule 3, disposés de part et d'autre de ce plan sensiblement à égale distance de celui-ci, ces deux plans parallèles étant en outre situés de part et d'autre de la tige de piston 5, de façon à permettre aux aiguilles 7 d'occuper leur position initiale rentrée en se croisant dans la partie d'extrémité 4 de la canule 3. Les aiguilles 7 sont situées au-dessus de la lame 8 en considérant la figure 2. Chaque aiguille 7 se termine par une partie pointue en forme de crochet 7a.

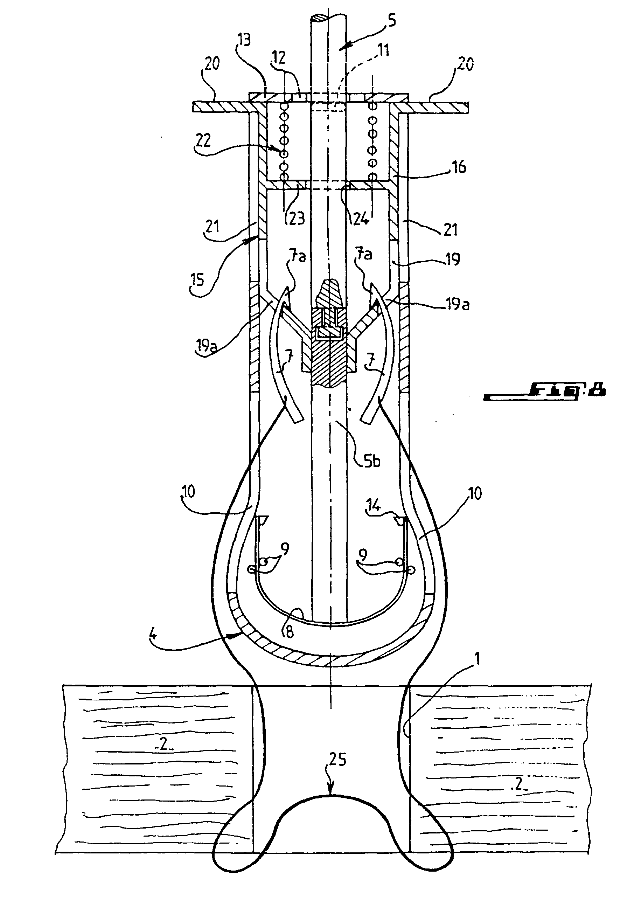

L'instrument comprend également un moyen 15 d'extraction des aiguilles 7 monté coulissant dans la canule 3 concentriquement à la tige 5 et pouvant être manoeuvré de l'extérieur pour le déplacer d'une position basse dans la partie d'extrémité 4 de la canule 3, comme représenté aux figures 1 à 4, à une position haute en partie supérieure de la canule 3 représentée en figure 8 de façon à saisir simultanément, lors de ce déplacement, les deux aiguilles 7 préalablement introduites dans la paroi abdominale 2 en pénétrant obliquement l'une vers l'autre dans l'orifice 1 et la canule 3 au travers des fenêtres longitudinales 10 et à extraire les aiguilles 7 de la lame flexible 8 pour les acheminer vers le haut dans la canule 3.

Le moyen d'extraction 15 comprend ainsi une partie supérieure cylindrique 16 pouvant coulisser le long de la surface latérale interne correspondante de la canule 3, une partie d'extrémité inférieure cylindrique 17 de plus petit diamètre dans laquelle est montée à coulissement la partie inférieure 5b de la tige 5, et une partie intermédiaire de liaison constituée de deux parois opposées 18 situées de part et d'autre de la tige 5, chaque paroi 18 comportant une partie droite 18a parallèle à la tige 5 et une partie oblique 18b convergeant vers la partie d'extrémité inférieure 17. Chaque paroi 18 comporte une fenêtre longitudinale 19 permettant le passage de l'aiguille correspondante 7 et se terminant, au-dessus de la partie inférieure 17 du moyen d'extraction 15, par deux bords 19a en forme de V au fond duquel peut s'accrocher l'extrémité en forme de crochet 7a de l'aiguille 7 lors du déplacement du moyen d'extraction 15 vers le haut de la canule 3. Du fait que les aiguilles 7 sont décalées du plan médian longitudinal de la canule 3, les fenêtres 19 le sont également.

Pour permettre une rotation de la partie supérieure 5a de la tige 5 relativement à sa partie inférieure 5b lors de l'opération de déverrouillage de la tige 5 de la canule 3 à partir de sa position initiale représentée en figure 1, la partie inférieure 5b peut être cannelée en s'engageant à coulissement dans l'alésage cannelé correspondant de la partie inférieure 17 du moyen d'extraction 15. Cependant cette disposition n'est pas obligatoire, car l'ensemble constitué par l'extrémité de la tige 5, la lame 8 et les axes 9 maintient en rotation la partie inférieure 5b de la tige 5 relativement à la canule.

Le moyen d'extraction 15 comporte en outre deux pattes externes de préhension 20 diamétralement opposées solidaires de la partie supérieure cylindrique 16 en faisant radialement saillie au travers respectivement de deux fenêtres longitudinales 21 de la paroi latérale de la canule 3 et qui sont situées au-dessus des fenêtres 10 de passage des aiguilles 7.

Le moyen d'extraction 15 est rappelé vers sa position basse dans la canule 3 par un ressort hélicoïdal de compression 22 monté précontraint entre la paroi supérieure de fermeture 13 de la canule 3 et une paroi transversale interne 23 du moyen d'extraction 15 située dans la partie supérieure cylindrique 16 de celui-ci. La paroi 23 comporte un orifice central circulaire 24 à travers lequel passe la tige 5.

Un fil de suture 25 a ses deux extrémités libres solidaires respectivement des deux aiguilles 7 à l'opposé de leurs extrémités en forme de crochet 7a et a une longueur suffisante pour constituer une boucle 25a située, dès l'introduction de l'instrument dans la paroi abdominale 2, à l'extérieur de celle-ci et maintenue à l'extérieur jusqu'au retrait de l'instrument de la paroi 2. Chaque extrémité du fil 25 peut être fixée à l'aiguille métallique correspondante 7 par sertissage.

La partie inférieure 4 de la canule 3 présente une extrémité renflée dans laquelle se trouvent les axes 9 de support de la lame 8 de façon à être atraumatique.

Le fonctionnement de l'instrument ressort déjà de la description qui en a été faite ci-dessus et va être maintenant expliqué.

Dans les conditions initiales d'utilisation de l'instrument, le moyen d'extraction 15 occupe sa position basse dans la canule 3 par le ressort de rappel 22 et les deux pattes 20 en appui respectivement sur les deux bords inférieurs des deux fenêtres 21 de la canule 3, et la tige 15 est verrouillée à sa position la plus basse dans la canule 3 de façon à exercer sur la lame 8 une pression de maintien des extrémités de la lame 8 et des deux aiguilles 7 à l'intérieur de la partie d'extrémité 4 de la canule 3 comme représenté en figure 1.

L'instrument est ensuite introduit dans l'orifice 1 au travers de la paroi 2 jusqu'à ce que la partie d'extrémité inférieure 4 soit située dans l'abdomen en dessous de la paroi 2. Cette manoeuvre est effectuée immédiatement après l'extraction du trocart utilisé auparavant pour l'intervention chirurgicale par laparoscopie avec l'abdomen maintenu gonflé par le gaz pour éviter une lésion des organes internes. Lors de l'introduction de l'instrument au travers de la paroi abdominale 2, le chirurgien aura pris soin de maintenir la boucle 25a du fil 25 à l'extérieur du corps du patient avec les parties d'extrémité du fil passant dans l'orifice 1 et traversant respectivement les deux fenêtres 10 de la partie d'extrémité 4.

Ensuite, la tige 5 est déverrouillée de la canule 3 en tournant la partie supérieure 5a à l'aide de la poignée 6 jusqu'à ce que les deux ergots 11 arrivent en aplomb de la lumière 12 et le chirurgien exerce alors une traction sur la tige 5 pour déplacer vers le haut de la canule 3 le centre géométrique de la lame 8 et, par glissement de celle-ci entre les deux paires d'axes de support 9, faire basculer les deux extrémités de la lame 8 et les deux aiguilles 7 en les éloignant les unes des autres jusqu'à la position représentée en figure 2 à laquelle les deux aiguilles 7 se trouvent juste en dessous de la face interne de la paroi abdominale 2. Ainsi, les deux aiguilles 7 se sont déplacées dans leurs plans parallèles respectifs de leur position croisée de part et d'autre de la tige 5 représentée en figure 1 à leur position représentée en figure 2 en passant à travers les fenêtres 19 du moyen d'extraction 15 et les fenêtres 10 de la partie d'extrémité 4 de la canule 3.

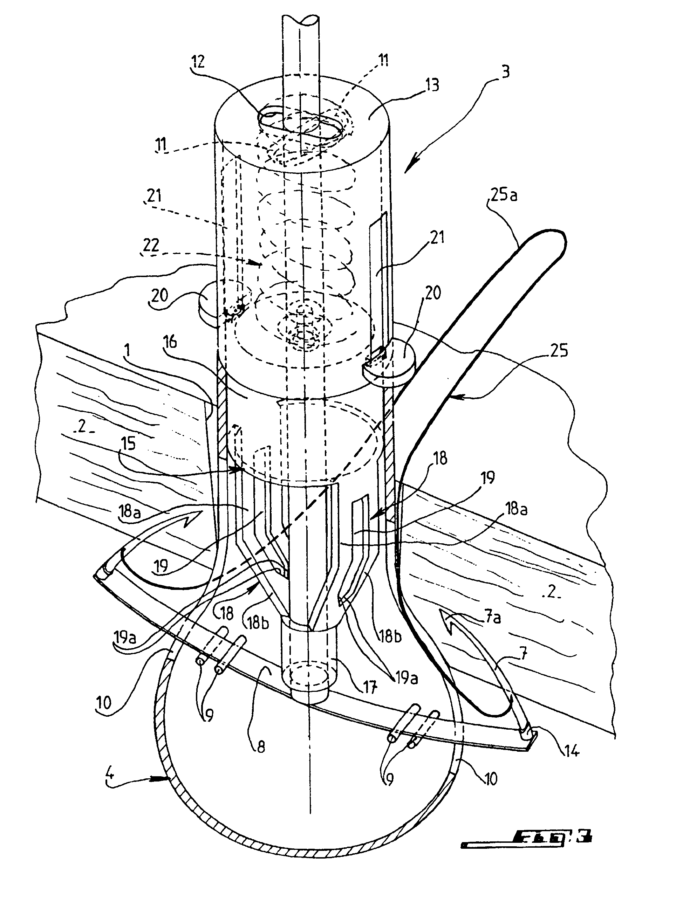

A partir de la position représentée en figure 2, le chirurgien exerce sur l'instrument un effort de traction vers le haut pour introduire les aiguilles 7 dans une partie d'épaisseur de la l'apanévrose de la paroi abdominale 2 comme représenté en figure 3.

Puis, le chirurgien enfonce à nouveau la tige 5 dans la canule 3 de façon que l'extrémité inférieure de la tige 5 plie par flexion la lame 8 qui glisse entre les deux paires d'axes 9 et les aiguilles 7 traversent complètement la partie d'épaisseur de la paroi abdominale 2 pour s'engager dans la canule 3 au travers des fenêtres 10 de la partie d'extrémité 4 de la canule 3 et des fenêtres 19 du moyen d'extraction 15 comme représenté en figure 4.

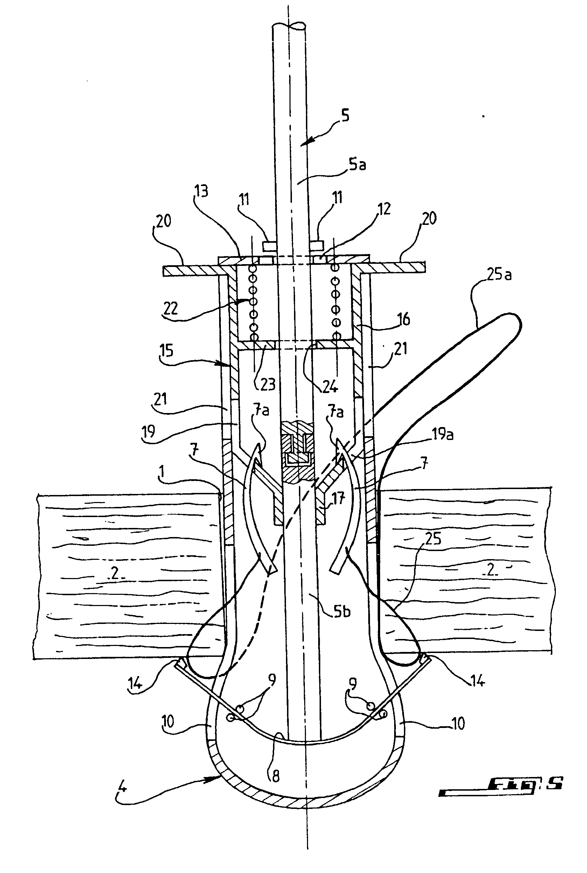

Le chirurgien déplace alors vers le haut de la canule 3 le moyen d'extraction 15 à l'aide des deux pattes externes 20 jusqu'à ce que les pattes 20 viennent en butée sur les bords supérieurs respectifs des deux fenêtres 21 de la canule 3 comme représenté en figure 5. Cette figure montre que pendant le déplacement vers le haut du moyen d'extraction 15, les bords inférieurs en forme de V des deux fenêtres 19 ont saisi les extrémités en forme de crochet 7a des aiguilles 7 pour les désengager de leurs embases respectives 14 avec les parties d'extrémité du fil 25 étant tirées par les aiguilles dans la canule 3 au travers des deux fenêtres 10. La figure 5 montre également que les deux embases 14 se trouvent pratiquement juste en dessous de la paroi abdominale 2.

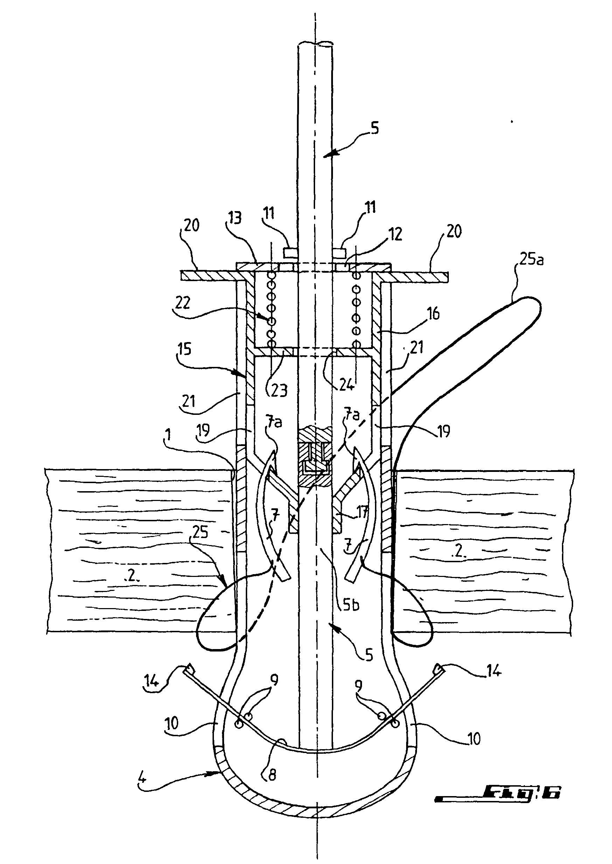

Ensuite, le chirurgien pousse l'instrument pour amener la partie d'extrémité inférieure 4 de la canule à une certaine distance de la paroi abdominale 2 (figure 6) et permettre, par poussée exercée sur la tige de piston 5, le repliement de la lame flexible 8 jusqu'à ce que les parties d'extrémité de celle-ci rentrent à nouveau, sans les aiguilles, dans la partie d'extrémité 4 comme représenté en figure 7. Le chirurgien manoeuvre la poignée 6 pour faire tourner la partie supérieure 5a de la tige 5 relativement à sa partie inférieure 5b et amener les deux ergots 11 en position de verrouillage de la tige 5 en dessous de la paroi supérieure de fermeture de la canule 3.

Enfin, l'instrument est complètement retiré de l'orifice 1 comme représenté en figure 8 et le fil 25 solidaire des aiguilles 7 est tiré de façon à continuer de passer à travers l'épaisseur de la paroi abdominale 2 suivant le même trajet que les aiguilles 7 jusqu'à ce que la boucle 25a soit située sensiblement au fond de l'orifice 1 au niveau de la face interne de la paroi abdominale 2. Le chirurgien n'a plus qu'alors à sectionner les deux parties d'extrémité du fil 25 et effectuer manuellement un noeud avec les deux brins du fil pour rapprocher et fermer les parois en vis-à-vis de l'orifice 1 comprenant l'apanévrose et le plan musculaire profond, avec tension de la partie de la boucle du fil sous la paroi 2.

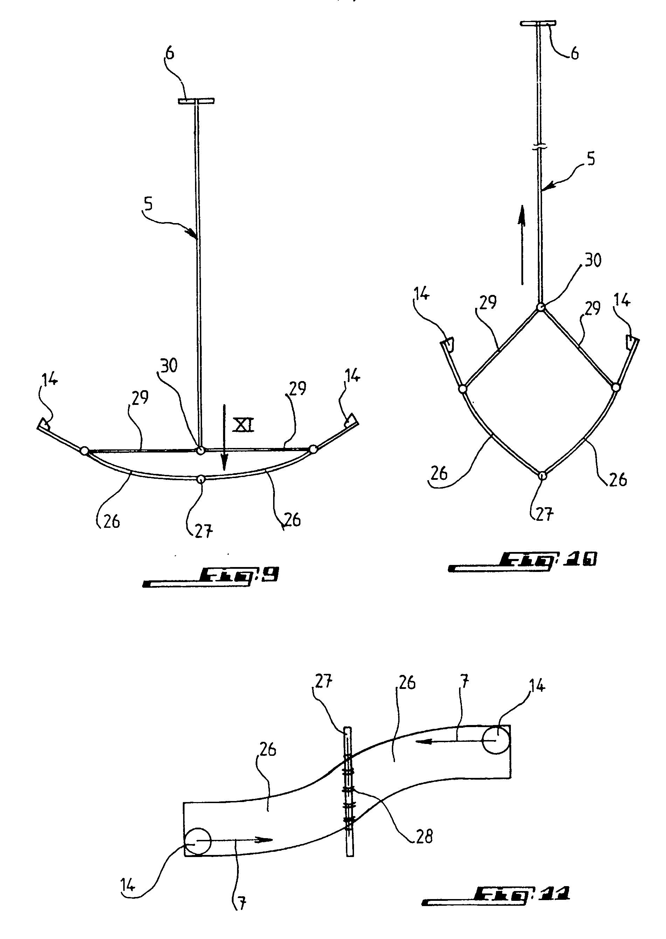

Les figures 9 à 11 représentent un autre mode de réalisation de l'organe de support des aiguilles 7 pouvant être utilisé à la place de celui constitué par la lame flexible 8 du premier mode de réalisation.

Selon ce second mode de réalisation, l'organe de support est constitué par deux bras 26 montés pivotants sur un axe central commun 27 solidaire du corps de la canule en partie inférieure 4 de celle-ci et s'étendant perpendiculairement au plan médian longitudinal de la canule. L'axe 27 peut être fixé par ses extrémités emmanchées à force respectivement dans deux perçages coaxiaux opposés réalisés dans la paroi latérale de la partie d'extrémité 4 de la canule 3 et les deux bras 26 sont montés pivotants sur l'axe 27 à la manière d'une charnière d'articulation 28.

Les deux bras 26 comportent, solidaires de leurs extrémités opposées, les deux embases 14 dans lesquelles sont amoviblement montées les deux aiguilles 7. Pour permettre aux deux embases 14 d'être situées dans deux plans écartés l'un de l'autre et parallèles au plan médian longitudinal de la canule 3, les deux bras 26 ont, vus de dessus, une configuration en forme de S allongé dont les branches incurvées sont situées de part et d'autre de l'axe 27 avec les deux embases situées sur une diagonale passant par le centre géométrique de l'axe 27.

La tige de piston 5, identique à la tige 5 du premier mode de réalisation, commande le pivotement simultané des deux bras 26 par l'intermédiaire de deux bras de traction 29 reliés d'une part l'un à l'autre à l'extrémité inférieure de la tige 5 par un axe d'articulation 30 solidaire de la tige parallèlement à l'axe de pivotement 27 et d'autre part à leurs extrémités opposées de façon articulée respectivement aux deux bras de support 26, au voisinage des embases 14 de support des aiguilles 7.

Lorsque l'instrument est introduit au travers de la paroi abdominale 2, les deux bras 26 occupent leur position représentée à la figure 10 à laquelle les deux bras sont relevés au-dessus de l'axe d'articulation 27 en définissant un angle aigu pour loger les parties d'extrémité des bras 26 et leurs aiguilles associées 7 dans la partie d'extrémité inférieure 4 de la canule 3.

Pour déployer les deux bras 26 à leur position d'ouverture en saillie au travers des fenêtres 10 de la partie inférieure 4 de la canule 3, le chirurgien exerce une poussée vers le bas de la canule de la tige de piston 5 de façon que les deux bras de traction 29 fassent pivoter les deux bras 26 autour de l'axe 27 en les écartant l'un de l'autre jusqu'à la position à laquelle les aiguilles 7 se trouvent en dessous de la paroi 2 comme représenté en figure 2.

Autrement, le principe de fonctionnement de l'instrument muni de l'organe de support selon le second mode de réalisation est identique à celui décrit précédemment en référence au premier mode de réalisation, mis à part qu'il faut exercer un effort de poussée sur la tige de piston 5 pour déployer les aiguilles à leur position en saillie en dehors de la canule 3 et tirer sur la tige 5 pour replier à nouveau en les rapprochant l'un de l'autre les deux bras 26 et les loger, sans les aiguilles, dans la partie d'extrémité 4 de la canule 3 avant extraction complète de l'instrument de l'orifice 1. L'autre différence existant relativement au premier mode de réalisation est que le verrouillage de la tige 5 notamment à la position de logement des bras 26 et des aiguilles 7 dans la partie d'extrémité 4 avant introduction de la canule dans la paroi 2 s'effectue en amenant les deux ergots de verrouillage 11 sur la paroi supérieure de fermeture de la canule 3 transversalement à la lumière 12 et, pour faciliter le déploiement des deux bras 26 à leur position représentée en figure 9, il est possible de prévoir un ressort de torsion enroulé sur l'axe de pivotement 27 et exerçant sur les deux bras 26 un effort élastique tendant à les écarter l'un de l'autre et à les maintenir à leur position déployée.

L'instrument conforme à l'invention permet de fermer efficacement l'orifice de passage du trocart et de suturer le muscle et l'aponévrose de la paroi abdominale d'un patient.

A titre d'indication, l'instrument peut avoir une longueur d'environ 20 cm avec un diamètre maximal externe de 12 mm au niveau de sa partie renflée, le diamètre de la canule sur ses deux tiers de sa longueur pouvant être de 10 mm. En position en saillie de la lame flexible 8 ou des bras 26 de support des aiguilles 7, leurs extrémités peuvent déborder de la partie d'extrémité inférieure 4 de la canule 3 de 5 mm à 7 mm de part et d'autre de celle-ci. Bien entendu, ces dimensions sont données à titre d'exemple non limitatif.

## Revendications

1. Instrument permettant de fermer par suture sous-cutanée un orifice (1) réalisé dans la paroi abdominale (2) d'un patient pour le passage d'un trocart ayant servi à une intervention chirurgicale par laparoscopie, comprenant une canule rigide cylindrique (3) dont une partie d'extrémité (4) peut être introduite dans l'orifice (1) ; un piston (5) s'étendant coaxialement dans la canule (3) et actionnable de l'extérieur par un opérateur ; deux aiguilles (7) amoviblement fixées aux extrémités diamétralement opposées d'un organe de support (8 ; 26) lui-même supporté dans la partie d'extrémité (4) de la canule (3), **caractérisé en ce que** l'organe de support (8;26) peut occuper, sous l'action du piston (5), une position ployée à laquelle les aiguilles (7) sont rentrées dans la partie d'extrémité (4) de la canule et une position déployée après introduction de la canule (3) dans l'orifice (1) et à laquelle les aiguilles (7) font complètement saillie de la canule en-dessous de la paroi abdominale (2) au travers respectivement de deux fenêtres longitudinales (10) de la paroi latérale de la canule (3) en étant inclinées relativement à l'axe longitudinal de la canule (3) de part et d'autre de celui-ci et dirigées l'une vers l'autre; un moyen (15) d'extraction des aiguilles (7) monté coulissant dans la canule (3) concentriquement au piston (5) et manoeuvrable par l'opérateur pour le déplacer d'une position basse dans la partie d'extrémité (4) de la canule (3) à une position haute dans la partie d'extrémité supérieure de celle-ci de façon à saisir simultanément, lors de ce déplacement, les deux aiguilles (7) préalablement introduites dans la paroi abdominale (2) en pénétrant obliquement l'une vers l'autre dans l'orifice (1) et la canule (3) au travers desdites fenêtres longitudinales (10), et à les extraire de l'organe de support (8 ; 26) pour les acheminer dans la canule (3) avec passage concomitant d'un fil de suture (25) dans la paroi abdominale (2) et la canule (3), le fil (25) ayant ses extrémités solidaires respectivement des deux aiguilles (7) et définissant une boucle (25a) située à l'extérieur de la canule en traversant l'orifice
(1) à l'extérieur de la paroi abdominale (2), les extrémités du fil (25) pouvant être ensuite saisies et sectionnées par l'opérateur, après retrait complet de la canule (3) de l'orifice (1), pour fermer l'orifice par la confection d'un noeud avec les deux brins de fil (25).

2. Instrument selon la revendication 1, **caractérisé en ce que** le piston est une tige rigide centrale (5) à coulissement guidé dans le moyen d'extraction (15) et dont l'extrémité inférieure est reliée à l'organe de support (8 ; 26) des aiguilles (7) et la partie d'extrémité supérieure (5a) traverse une paroi transversale supérieure (13) de fermeture de la canule (3), la tige (5) pouvant être axialement retenue dans la canule (3) par un moyen de verrouillage (11) manuellement déverrouillable de façon à exercer sur l'organe de support (8 ; 26) une force axiale de rétraction des aiguilles (7) dans la canule (3) pour permettre l'introduction de celle-ci dans l'orifice (1), l'opérateur pouvant ensuite déverrouiller le moyen de verrouillage (11) pour déplacer la tige (5) dans un sens provoquant le déploiement de l'organe de support (8 ;26) et la sortie des aiguilles (7) de la canule (3).

3. Instrument selon la revendication 2, **caractérisé en ce que** le moyen d'extraction (15) comprend une partie supérieure cylindrique de coulissement (16) dans la canule (3), une partie d'extrémité inférieure cylindrique (17) de plus petit diamètre dans laquelle est montée à coulissement la partie d'extrémité inférieure (5b) de la tige (5) et une partie intermédiaire de liaison constituée notamment de deux parois obliques (18b) convergeant vers la partie d'extrémité inférieure (17) et comprenant chacune une fenêtre longitudinale (19) permettant, en position basse du moyen d'extraction (15), le passage de l'aiguille correspondante (7) à sa position sortie en saillie de la canule (3), chaque fenêtre (19) se terminant au-dessus de la partie inférieure (17) du moyen d'extraction (15) par deux bords (19a) en forme de V dans lequel peut s'accrocher l'extrémité en forme de crochet (7a) de l'aiguille (7) lors du déplacement du moyen d'extraction (15) vers la partie supérieure de la canule (3).

4. Instrument selon la revendication 3, **caractérisé en ce que** le moyen d'extraction (15) comprend deux pattes externes de préhension (20) diamétralement opposées solidaires de la partie supérieure 16) de ce moyen en faisant radialement saillie au travers respectivement de deux fenêtres longitudinales (21) de la paroi latérale de la canule (3) situées au-dessus des fenêtres de passage (10) des aiguilles (7) et **en ce qu'**un ressort (22) est monté précontraint entre le moyen d'extraction (15) et la canule (3) pour rappeler le moyen d'extraction (15) à sa position basse.

5. Instrument selon l'une des revendications précédentes, **caractérisé en ce que** les aiguilles (7) sont courbées et situées respectivement dans deux plans parallèles au plan médian longitudinal de la canule (3), disposés de part et d'autre de ce plan médian à égale distance de celui-ci de façon à permettre aux aiguilles (7) d'occuper leur position rentrée en se croisant dans la partie d'extrémité (4) de la canule (3).

6. Instrument selon l'une des revendications précédentes, **caractérisé en ce que** les aiguilles (7) sont montées amoviblement par leurs extrémités opposées à celles en forme de crochet (7a) respectivement dans deux embases (14) à section transversale circulaire, triangulaire, ou autre, et solidaires des extrémités de l'organe de support (8 ; 26), l'axe de chaque embase (14) étant incliné relativement au plan de support d'extrémité correspondante de l'organe de support pour faciliter l'extraction des aiguilles (7) lors de la remontée du moyen d'extraction (15) dans la canule (3).

7. Instrument selon l'une des revendications précédentes, **caractérisé en ce que**, lorsque les aiguilles (7) occupent leur position sortie de la canule (3), cette dernière est déplacée par traction pour faire pénétrer les aiguilles (7) dans la paroi abdominale (2) et le piston (5) est actionné dans un sens permettant de rétracter les aiguilles (7) vers la canule (3) et de faire pénétrer les extrémités de celles-ci dans l'orifice (1) et dans la canule (3) au travers des deux fenêtres (10) diamétralement opposées de celle-ci.

8. Instrument selon la revendication 5, **caractérisé en ce que** les deux plans contenant respectivement les deux aiguilles (7) sont situés de part et d'autre de la tige (5) du piston.

9. Instrument selon l'une des revendications 2 à8, **caractérisé en ce que** l'organe de support est constitué par une lame flexible (8) portant à ses extrémités les deux aiguilles (7) et maintenue dans la partie inférieure (4) de la canule (3) perpendiculairement au plan médian longitudinal de cette dernière par deux paires d'axes de support (9) parallèles à la lame (8), disposées de part et d'autre du plan médian transversal de la lame (8) symétriquement à celui-ci et solidaires du corps de la canule (3) perpendiculairement à son plan médian longitudinal, les deux axes (9) de chaque paire étant situés de chaque côté de la lame (8) à proximité immédiate de celle-ci pour modifier par flexion le rayon de courbure de la lame (8) par glissement de celle-ci entre les axes de support (9) sous l'action de la tige de piston (5), dont l'extrémité inférieure est solidaire du centre géométrique de la lame (8), et déplacer ce centre vers le bas ou vers le haut de la canule (3) pour sortir ou rentrer, et ultérieurement rentrer partiellement, les aiguilles (7) de ou dans la canule (3).

10. Instrument selon l'une des revendications 2 à 8, **caractérisé en ce que** l'organe de support est constitué par deux bras (26) montés pivotant sur un axe central commun (27) solidaire du corps de la canule (3) en partie inférieure (4) de celle-ci et s'étendant perpendiculairement au plan médian longitudinal de la canule, les extrémités opposées des deux bras (26) portant les aiguilles (7), et **en ce que** le piston (5) commande le pivotement simultané des deux bras (26) en le déplaçant vers le bas ou vers le haut pour sortir ou rentrer les aiguilles (7) de ou dans la canule (3) par l'intermédiaire de deux bras de traction (29) reliés d'une part l'un à l'autre à l'extrémité inférieure de la tige du piston (5) par un axe d'articulation (30) solidaire du piston parallèlement à l'axe de pivotement (27) des bras de support (26) des aiguilles (7) et d'autre part à leurs extrémités opposées de façon articulée respectivement aux deux bras de support (26), au voisinage des aiguilles (7).

11. Instrument selon la revendication 9 ou 10, **caractérisé en ce que** le moyen de verrouillage de la tige (5) à sa position de ploiement de l'organe de support (8 ; 26) comprend deux ergots diamétralement opposés (11) solidaires de la partie supérieure (5a) de la tige (5) en faisant radialement saillie de celle-ci et venant en appui sous ou sur la paroi transversale de fermeture (13) de la canule (3) suivant que l'organe de support est constitué par la lame flexible (8) ou les deux bras pivotants (26), cette paroi (13) comportant un trou oblong (12) défini de part et d'autre de l'orifice central de passage de la tige (5) et permettant le passage des deux ergots (11) à travers celui-ci par rotation de la partie supérieure (5a) de la tige (5) relativement à sa partie inférieure (5b) pour déverrouiller la tige (5) et la déplacer axialement dans la canule (3).

## Claims

1. Instrument for sealing by subcutaneous suture of an opening (1) made in the abdominal wall (2) of a patient to pass through a trocar used for surgical intervention by laparoscopy, consisting of a rigid cylindrical cannula (3), one end of which (4) can be inserted into the opening (1); a plunger (5) which extends coaxially into the cannula (3) and can be actuated from outside by an operator; two needles (7) removably fixed to the diametrically opposite ends of a support unit (8; 26) which is supported in turn in the end part (4) of the cannula (3), **characterised in that** when actuated by the plunger (5), the support unit (8; 26) can occupy a folded position in which the needles (7) are retracted in the end section (4) of the cannula and an extended position after insertion of the cannula (3) into the opening (1) in which the needles (7) project fully from the cannula below the abdominal wall (2) through two longitudinal windows (10) in the side wall of the cannula (3), are inclined relative to the longitudinal axis of the cannula (3) and on either side of it and are facing each other; a means (15) for extraction of the needles (7) which is mounted sliding in the cannula (3) concentrically with the plunger (5) and can be manoeuvred by the operator to move it from a lower position in the end section (4) of the cannula (3) to an upper position in its top section so as to grasp the two needles (7) previously inserted into the abdominal wall (2) simultaneously during the movement, by penetrating obliquely towards each other into the opening (1) and the cannula (3) through said longitudinal windows (10), and to extract them from the support unit (8; 26) and convey them into the cannula (3) and at the same time to thread a suture (25) into the abdominal wall (2) and the cannula (3), the thread (25) having each end joined to one of the two needles (7) to form a loop (25a) outside the cannula by passing through the opening (1) on the outside of the abdominal wall (2), the ends of the thread (25) then being picked up and severed by the operator, after complete withdrawal of the cannula (3) from the opening (1), to seal the opening by knotting the two strands of thread (25) together.

2. Instrument according to claim 1, **characterised in that** the plunger is a central rigid sliding rod (5) guided in the extraction means (15), the bottom of which is connected to the support unit (8; 26) for the needles (7) and the top of which (5a) passes through an upper transverse wall (13) for closure of the cannula (3), the rod (5) being able to be held axially in the cannula (3) by a locking means (11) which can be unlocked manually so as to exert an axial force on the support unit (8; 26) for retraction of the needles (7) in the cannula (3) so that it can be inserted into the opening (1) and the operator can then unlock the locking means (11) to move the rod (5) in a direction which causes extension of the support unit (8; 26) and emergence of the needles (7) from the cannula (3).

3. Instrument according to claim 2, **characterised in that** the extraction means (15) comprise an upper cylindrical sliding section (16) in the cannula (3), a lower cylindrical section (17) with a smaller diameter, slide mounted in which is the bottom part (5b) of the rod (5), and an intermediate link section formed mainly by two oblique walls (18b) converging towards the bottom section (17) and each containing a longitudinal window (19) which, in the lower position of the extraction means (15), allows the corresponding needle (7) to pass through to its emerged position projecting from the cannula (3), each window (19) terminating above the bottom section (17) of the extraction means (15) in two edges (19a) in the form of a V to which the end of the needle (7) in the form of a hook (7a) can be fastened during the movement of the extraction means (15) towards the top section of the cannula (3).

4. Instrument according to claim 1, **characterised in that** the extraction means (15) comprise two diametrically opposite external gripping attachments (20) joined to the top section (16) of said means, each projecting radially through one of two longitudinal windows (21) in the side wall of the cannula (3) arranged above windows (10) for the needles (7) to pass through and **in that** a spring (22) is mounted prestressed between the extraction means (15) and the cannula (3) to return the extraction means (15) to its lower position.

5. Instrument according to any of the preceding claims, **characterised in that** the needles (7) are curved and are located respectively in two parallel planes on the longitudinal median plane of the cannula (3), being arranged on either side of said median plane at an equal distance from it to allow the needles (7) to occupy their returned position by meeting in the lower section (4) of the cannula (3).

6. Instrument according to any of the preceding claims, **characterised in that** the needles (7) are each movably mounted by their ends opposite to the ends in hook form (7a) in one of two bases (14) with a circular, triangular or other section and are joined to the ends of the support unit (8; 26), the axis of each base (14) being inclined relative to the corresponding end support plane of the support unit to facilitate extraction of the needles (7) during the ascent of the extraction means (15) in the cannula (3).

7. Instrument according to any of the preceding claims, **characterised in that** when the needles (7) occupy their position of being emerged from the cannula (3), said cannula is moved by traction to push the needles (7) into the abdominal wall (2) and the plunger (5) is operated in a direction which retracts the needles (7) towards the cannula (3) and pushes their ends into the opening (1) and into the cannula (3) through the two windows (10) diametrically opposite it.

8. Instrument according to claim 5, **characterised in that** the two planes containing the two needles (7) are arranged on either side of the plunger rod (5).

9. Instrument according to any of claims 2 to 8, **characterised in that** the support unit consists of a flexible strip (8) carrying the two needles (7) at its ends and is held in the bottom section (4) of the cannula (3) perpendicular to its longitudinal median plane by two pairs of support pins (9) parallel to the strip (8), arranged on either side of the transverse median plane of the blade (8), symmetrical with it and joined to the body of the cannula (3) perpendicular to its longitudinal median plane, both pins (9) of each pair being located on each side of the strip (8) and immediately adjacent to it, to change the radius of curvature of the strip (8) by bending, by sliding it between the support pins (9) under the action of the plunger rod (5), the lower end of which is integral with the geometrical centre of the strip (8), and to move said centre towards the bottom or top of the cannula (3) to remove or return and later partially return the needles (7) from or to the cannula (3).

10. Instrument according to any of claims 2 to 8, **characterised in that** the support unit consists of two arms (26) mounted pivoting around a central common pin (27) joined to the body of the cannula (3) at its bottom section (4) and extending perpendicular to the longitudinal median plane of the cannula, whereby the opposite ends of the two arms (26) carry the needles (7), and **in that** the plunger (5) controls the simultaneous pivoting of the two arms (26) by moving them down or up to remove or return the needles (7) from or to the cannula (3) by means of two traction arms (29) connected to each other both at the bottom of the plunger rod (5) by a hinge pin (30) integral with the plunger parallel to the pivoting pin (27) of the support arms (26) of the needles (7) and also at their opposite ends so that both are hinged on the two support arms (26) adjacent to the needles (7).

11. Instrument according to claim 9 or 10, **characterised in that** the locking means of the rod (5) in its position of folding the support unit (8; 26) contains two diametrically opposite lugs (11) which are integral with the top section (5a) of the rod (5) and projects radially from it and is supported under or on the transverse closure wall (13) of the cannula (3) such that the support unit is formed by the flexible strip (8) or the two pivoting arms (26), said wall (13) containing a slot (12) bounded on either side by the central through hole of the rod (5) and allows the two lugs (11) to pass through it by rotation of the top section (5a) of the rod (5) relative to its bottom section (5b) to unlock the rod (5) and move it axially in the cannula (3).

## Patentansprüche

1. Instrument, das es ermöglicht, mit einer Subkutannaht eine Öffnung (1) zu verschließen, die in der Bauchwand (2) eines Patienten zum Durchführen eines Trokars hergestellt wurde, der bei einem laparoskopischen chirurgischen Eingriff verwendet wurde, das eine walzenförmige starre Kanüle (3) umfasst, von der ein Endabschnitt (4) in die Öffnung (1) eingeführt werden kann, einen Kolben (5), der sich koaxial in der Kanüle (3) erstreckt und von einer Bedienperson von außen betätigt werden kann, zwei Nadeln (7), die lösbar an den diametral entgegengesetzten Enden eines Halteelements (8; 26) befestigt sind, das selbst im Endabschnitt (4) der Kanüle (3) gehalten ist, **dadurch gekennzeichnet, dass** das Halteelement (8; 26) unter der Einwirkung des Kolbens (5) eine gebogene Position einnehmen kann, in der die Nadeln (7) in den Endabschnitt (4) der Kanüle eingezogen sind, und eine ausgebreitete Position nach dem Einführen der Kanüle (3) in die Öffnung (1) und in der die Nadeln (7) unter der Bauchwand (2) vollständig jeweils durch die beiden längs laufenden Schlitzlöcher (10) der Seitenwand der Kanüle (3) aus der Kanüle hervorragen und dabei relativ zur Längsachse der Kanüle (3) auf beiden Seiten derselben geneigt und aufeinander zu gerichtet sind, ein Mittel (15) zum Herausziehen der Nadeln (7), das verschiebbar in der Kanüle (3) konzentrisch zum Kolben (5) befestigt ist und von der Bedienperson betätigt werden kann, um es von einer tiefen Position im Endabschnitt (4) der Kanüle (3) in eine hohe Position im oberen Endabschnitt derselben zu verschieben, damit es während dieser Verschiebung gleichzeitig die beiden Nadeln (7) greift, die zuvor in die Bauchwand (2) eingeführt wurden, indem sie schräg zueinander durch die längs laufenden Schlitzlöcher (10) hindurch in die Öffnung (1) und die Kanüle (3) eindringen, und damit es sie aus dem Halteelement (8; 26) herauszieht, um sie in die Kanüle (3) zu befördern, bei gleichzeitigem Führen eines Fadens (25) in die Bauchwand (2) und die Kanüle (3), wobei die Enden des Fadens (25) jeweils einstückig mit den beiden Nadeln (7) sind und der Faden eine Schlaufe (25a) definiert, die sich außerhalb der Kanüle befindet und die Öffnung (1) außerhalb der Bauchwand (2) durchquert, wobei die Enden des Fadens (25) anschließend, nach dem vollständigen Herausziehen der Kanüle (3) aus der Öffnung (1), von der Bedienperson gegriffen und durchgeschnitten werden können, um die Öffnung durch die Anfertigung eines Knotens mit den beiden Einzelfäden (25) zu verschließen.

2. Instrument nach Anspruch 1, **dadurch gekennzeichnet, dass** der Kolben eine mittige starre verschiebbare Stange (5) ist, die im Herausziehmittel (15) geführt ist und deren unteres Ende mit dem Halteelement (8; 26) für die Nadeln (7) verbunden ist, und der obere Endabschnitt (5a) durchquert eine obere Querwand (13) zum Verschließen der Kanüle (3), wobei die Stange (5) in Achsrichtung in der Kanüle (3) von einem Verriegelungsmittel (11) gehalten werden kann, das manuell gelöst werden kann, um auf das Halteelement (8; 26) eine axiale Kraft zum Zurückziehen der Nadeln (7) in die Kanüle (3) auszuüben, um das Einführen derselben in die Öffnung (1) zu ermöglichen, wobei die Bedienperson anschließend das Verriegelungsmittel (11) lösen kann, um die Stange (5) in eine Richtung zu verschieben, durch die das Ausbreiten des Halteelements (8; 26) und das Heraustreten der Nadeln (7) aus der Kanüle (3) bewirkt wird.

3. Instrument nach Anspruch 2, **dadurch gekennzeichnet, dass** das Herausziehmittel (15) einen walzenförmigen oberen Abschnitt zum Verschieben (16) in der Kanüle (3) umfasst, einen walzenförmigen unteren Endabschnitt (17) mit einem geringeren Durchmesser, in dem verschiebbar der untere Endabschnitt (5b) der Stange (5) befestigt ist, und einen Verbindungszwischenabschnitt, der insbesondere aus zwei schrägen Wänden (18b) besteht, die zum unteren Endabschnitt (17) zusammenlaufen und jeweils ein längs laufendes Schlitzloch (19) umfassen, das in der tiefen Position des Herausziehmittels (15) den Übergang der entsprechenden Nadel (7) in die vorstehende Position ermöglicht, in der sie aus der Kanüle (3) hervorragt, wobei jedes Schlitzloch (19) über dem unteren Abschnitt (17) des Herausziehmittels (15) mit zwei V-förmigen Rändern (19a) endet, in denen sich das hakenförmige Ende (7a) der Nadel (7) beim Verschieben des Herausziehmittels (15) in Richtung des oberen Abschnitts der Kanüle (3) festhaken kann.

4. Instrument nach Anspruch 3, **dadurch gekennzeichnet, dass** das Herausziehmittel (15) zwei äußere, diametral entgegengesetzte Ansatzstücke (20) zum Greifen umfasst, die einstückig mit dem oberen Abschnitt (16) dieses Mittels sind und radial jeweils durch die beiden längs laufenden Schlitzlöcher (21) der Seitenwand der Kanüle (3) hindurch, die sich über den Durchgangsschlitzlöchern (10) für die Nadeln (7) befinden, ragen, und **dadurch**, dass eine Feder (22) vorgespannt zwischen dem Herausziehmittel (15) und der Kanüle (3) montiert ist, um das Herausziehmittel (15) zurück in seine tiefe Position zu bringen.

5. Instrument nach einem der vorhergehenden Ansprüche, **dadurch gekennzeichnet, dass** die Nadeln (7) gekrümmt sind und sich jeweils in zwei Ebenen befinden, die parallel zur längs verlaufenden Mittelebene der Kanüle (3) verlaufen und auf beiden Seiten dieser Mittelebene mit dem gleichen Abstand zu derselben angeordnet sind, damit die Nadeln (7) ihre eingezogene Position einnehmen können, in der sie sich im Endabschnitt (4) der Kanüle (3) kreuzen.

6. Instrument nach einem der vorhergehenden Ansprüche, **dadurch gekennzeichnet, dass** die Nadeln (7) an ihren Enden gegenüber den hakenförmigen Enden (7a) jeweils in zwei Ansätzen (14) mit einem kreisförmigen, dreieckigen oder einem anderen Querschnitt, die einstückig mit den Enden des Halteelements (8; 26) sind, lösbar befestigt sind, wobei die Achse jedes Ansatzes (14) relativ zur Halteebene des entsprechenden Endes des Halteelements geneigt ist, um das Herausziehen der Nadeln (7) bei der Aufwärtsbewegung des Herausziehmittels (15) in der Kanüle (3) zu vereinfachen.

7. Instrument nach einem der vorhergehenden Ansprüche, **dadurch gekennzeichnet, dass**, wenn die Nadeln (7) die Position einnehmen, in der sie aus der Kanüle (3) herausgetreten sind, letztere durch Ziehen verschoben wird, damit die Nadeln (7) in die Bauchwand (2) eindringen, und der Kolben (5) in einer Richtung betätigt wird, die es ermöglicht, dass die Nadeln (7) in Richtung der Kanüle (3) zurückgezogen werden und die Enden derselben in die Öffnung (1) und in die Kanüle (3) durch die beiden diametral entgegengesetzten Schlitzlöcher (10) derselben eindringen.

8. Instrument nach Anspruch 5, **dadurch gekennzeichnet, dass** sich die beiden Ebenen, die jeweils die beiden Nadeln (7) enthalten, auf beiden Seiten der Kolbenstange (5) befinden.

9. Instrument nach einem der Ansprüche 2 bis 8, **dadurch gekennzeichnet, dass** das Halteelement von einem flexiblen Plättchen (8) gebildet wird, das an seinen Enden die beiden Nadeln (7) trägt und im unteren Abschnitt (4) der Kanüle (3) senkrecht zur längs verlaufenden Mittelebene der letzteren parallel zum Plättchen (8) von zwei Paaren von Haltestiften (9) gehalten wird, die auf beiden Seiten der quer verlaufenden Mittelebene des Plättchens (8) symmetrisch zu dieser und einstückig mit dem Körper der Kanüle (3) senkrecht zu ihrer längs verlaufenden Mittelebene angeordnet sind, wobei sich die beiden Stifte (9) jedes Paars auf jeder Seite des Plättchens (8) in der unmittelbaren Nähe desselben befinden, um durch Biegen den Krümmungsradius des Plättchens (8) durch Gleiten desselben zwischen den Haltestiften (9) unter dem Einfluss der Kolbenstange (5) zu verändern, deren unteres Ende einstückig mit dem geometrischen Mittelpunkt des Plättchens (8) ist, und diesen Mittelpunkt zum Unterteil oder Oberteil der Kanüle (3) hin zu verschieben, damit die Nadeln (7) aus der Kanüle (3) treten oder in die Kanüle (3) eingezogen werden und später teilweise eingezogen werden.

10. Instrument nach einem der Ansprüche 2 bis 8, **dadurch gekennzeichnet, dass** das Halteelement von zwei Armen (26) gebildet wird, die schwenkbar auf einer gemeinsamen Mittelachse (27) befestigt sind, die einstückig mit dem Körper der Kanüle (3) im unteren Abschnitt (4) derselben ist und sich senkrecht zur längs verlaufenden Mittelebene der Kanüle erstreckt, wobei die entgegengesetzten Enden der beiden Arme (26) die Nadeln (7) tragen, und **dadurch**, dass der Kolben (5) die gleichzeitige Schwenkbewegung der beiden Arme (26) steuert, wenn er nach unten oder nach oben verschoben wird, damit die Nadeln (7) aus der Kanüle (3) treten oder in die Kanüle (3) eingezogen werden, über zwei Zugarme (29), die einerseits am unteren Ende der Kolbenstange (5) über eine Drehachse (30) aneinander befestigt sind, die einstückig mit dem Kolben parallel zur Schwenkachse (27) der Haltearme (26) für die Nadeln (7) ist, und andererseits an ihren entgegengesetzten Enden in der Nähe der Nadeln (7) gelenkig jeweils an den beiden Haltearmen (26) befestigt sind.

11. Instrument nach Anspruch 9 oder 10, **dadurch gekennzeichnet, dass** das Mittel zum Blockieren der Stange (5) in der Position, in der das Halteelement (8; 26) gebogen ist, zwei diametral entgegengesetzte Vorsprünge (11) umfasst, die einstückig mit dem oberen Abschnitt (5a) der Stange (5) sind und radial von diesem hervorstehen und unter oder auf der Querwand zum Verschließen (13) der Kanüle (3) anliegen, je nachdem, ob das Halteelement von dem flexiblen Plättchen (8) oder den beiden Schwenkarmen (26) gebildet wird, wobei diese Wand (13) ein Langloch (12) umfasst, das auf beiden Seiten der mittigen Durchgangsöffnung der Stange (5) definiert ist und das Hindurchführen der beiden Vorsprünge (11) durch das Loch hindurch durch Drehen des oberen Abschnitts (5a) der Stange (5) relativ zu ihrem unteren Abschnitt (5b) ermöglicht, um die Stange (5) zu lösen und sie in Achsrichtung in der Kanüle (3) zu verschieben.
